# EUROPEAN PATENT APPLICATION

(11) **EP 2 129 169 A2**
(43) Date of publication of application: **02.12.2009**
(21) Application number: 09161477.6
(22) Date of filing: 29.05.2009
(51) Int. Cl.: H04R 25/00

(54) **Measurement of sound pressure level and phase at eardrum by sensing eardrum vibration**

(30) Priority: 30.05.2008 US 130764
(71) Applicant: Starkey Laboratories, Inc., Eden Prairie, MN 55344 (US)
(72) Inventor: Zhang, Tao, Eden Prairie, MN 55344 (US)
(74) Representative: Fox, Nicholas Russell Philip

(57) **Abstract**

A hearing assistance device for measuring sound pressure at a tympanic membrane of a user's ear, the device comprising a housing adapted to be worn at least partially in an ear canal of the user a laser source coupled to the housing and adapted to project a beam of laser energy at the tympanic membrane a sensor for receiving reflected laser energy directed at the tympanic membrane, and a processor connected to the sensor and adapted to estimate the sound pressure level and phase at the tympanic membrane from a signal generated from the sensor. Additional examples provide a hearing device for measuring sound pressure at the tympanic membrane using ultrasonic signals. Further examples provide a hearing device for measuring sound pressure at the tympanic membrane using magnetic sources and sensors.

## Description

### Field of Technology

This application relates generally to hearing assistance devices and more particularly to a system for estimating sound pressure level and phase at a wearer's eardrum by sensing eardrum vibration.

### Background

Hearing assistance devices, including hearing aids, are electronic devices that provide signal processing functions such as wide dynamic range compression and output compression limiting control. In many hearing assistance devices these and other functions can be programmed to fit the requirements of individual users. Performance of a user's hearing assistance device, while the device is in the user's ear, is difficult to verify. The expense of measurement equipment, the time it takes to make the measurements, and the perceived complexity of the procedure, have all proven to be obstacles to widespread use of such measurements. However, such measurements may enable better programming of a user's hearing assistance device because each user's ear is different.

### Summary

This document provides method and apparatus for estimating the sound field at a user's tympanic membrane, or eardrum. One example provides a hearing assistance device, including a laser based eardrum vibration detector and processor for estimating the sound level and phase at the wearer's eardrum. One example provides a hearing assistance device, including an ultrasonic based eardrum vibration detector and processor for estimating the sound level and phase at the wearer's eardrum. The sound pressure estimates may be used to adjust the parameters of the hearing assistance device to provide for better performance of the device or comfort of the wearer. One example provides a method of estimating the sound field near a user's eardrum including attaching a magnetic material to the eardrum, inserting a probe with a pickup coil into the user's ear canal, capturing a signal indicative of eardrum movement using the pickup coil and processing the signal to provide an estimate of the sound level and phase at the eardrum.

This Summary is an overview of some of the teachings of the present application and is not intended to be an exclusive or exhaustive treatment of the present subject matter. Further details about the present subject matter are found in the detailed description and the appended claims. The scope of the present invention is defined by the appended claims.

### Brief Description of the Drawings

FIG. 1A illustrates a hearing assistance device with a vibration detector according to one embodiment of the present subject matter.

FIG. 1B illustrates is a block diagram of a hearing assistance device with ear drum vibration processing according to one embodiment of the present subject matter.

FIG. 1C is a block diagram of a hearing assistance device with ear drum vibration processing according to one embodiment of the present subject matter.

FIG. 2 illustrates a hearing assistance device with an eardrum vibration detector according to one embodiment of the present subject matter.

FIG. 3 illustrates a hearing assistance device with an eardrum vibration detector according to one embodiment of the present subject matter.

FIG. 4 illustrates an end view of a hearing assistance device for sensing eardrum vibration according to one embodiment of the present subject matter.

FIG. 5 illustrates an end view of a hearing assistance device for sensing eardrum vibration to estimate a sound field at the eardrum of a user according to one embodiment of the present subject matter.

FIG. 6 illustrates a hearing assistance device having a behind-the-ear (BTE) housing with ear drum vibration sensing to estimate a sound field at the eardrum of a user according to one embodiment of the present subject matter.

FIG. 7A illustrates a block diagram of a hearing assistance device having a behind-the-ear (BTE) housing with ear drum vibration sensing to estimate a sound field at the eardrum of a user according to one embodiment of the present subject matter.

FIG. 7B illustrates a block diagram of a hearing assistance device having a behind-the-ear (BTE) housing to estimate a sound field at the eardrum of a user according to one embodiment of the present subject matter.

FIG. 8A illustrates a hearing assistance device having magnetic wave detection electronics to estimate a sound field at the eardrum of a user according to one embodiment of the present subject matter.

FIG. 8B illustrates a block diagram of a hearing assistance device having a behind-the-ear (BTE) housing to estimate a sound field at the eardrum of a user according to one embodiment of the present subject matter.

FIG. 9 illustrates a magnetic wave probe for detecting eardrum vibration of a user and estimating the sound field at the user's eardrum according to one embodiment of the present subject matter.

FIG. 10 illustrates a flow diagram for estimating sound level and phase at the eardrum of a user according to one embodiment of the present subject matter.

### Detailed Description

The following detailed description of the present invention refers to subject matter in the accompanying drawings which show, by way of illustration, specific aspects and embodiments in which the present subject matter may be practiced. These embodiments are described in sufficient detail to enable those skilled in the art to practice the present subject matter. References to "an", "one", or "various" embodiments in this disclosure are not necessarily to the same embodiment, and such references contemplate more than one embodiment. The following detailed description is, therefore, not to be taken in a limiting sense, and the scope is defined only by the appended claims, along with the full scope of legal equivalents to which such claims are entitled.

The sound field in an individual's ear canal is generally more uniform when subjected to low frequency sound because of the longer wavelength. Because of the uniformity, it is assumed that sound pressure levels and phase sensed near the eardrum provide an accurate measure of the sound pressure level and phase at the eardrum. However, the sound field becomes less uniform and more complex as the eardrum and ear canal are subjected to higher frequency sounds. It is risky and uncomfortable, to measure the sound pressure level at the eardrum by placing a sensor very close to the eardrum. Furthermore, it is difficult to predict the sound pressure level at the eardrum without placing a sensor very close to the eardrum.

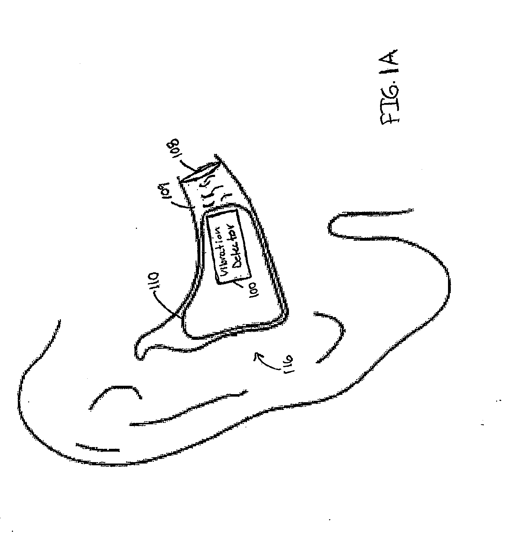

FIG. 1A illustrates a hearing assistance device with a vibration detector according to one embodiment of the present subject matter. The hearing assistance device 116 is adapted to be worn in a user's ear canal 109 and includes a housing 110 enclosing an eardrum vibration detector 100. The vibration detector senses vibration of the user's ear drum 108 to estimate sound pressure at or very close to the eardrum 108.
In various embodiments, the detector senses ear drum vibration using a magnetic media attached to the eardrum. In various embodiments, the vibration detector senses ear drum vibration using detection signals emitted from the detector and reflected back to the detector by the user's ear drum, or tympanic membrane. In various embodiments, the detector includes a laser source to emit a detection signal. In various embodiments, the detector uses an ultrasonic emitter to emit a detection signal.

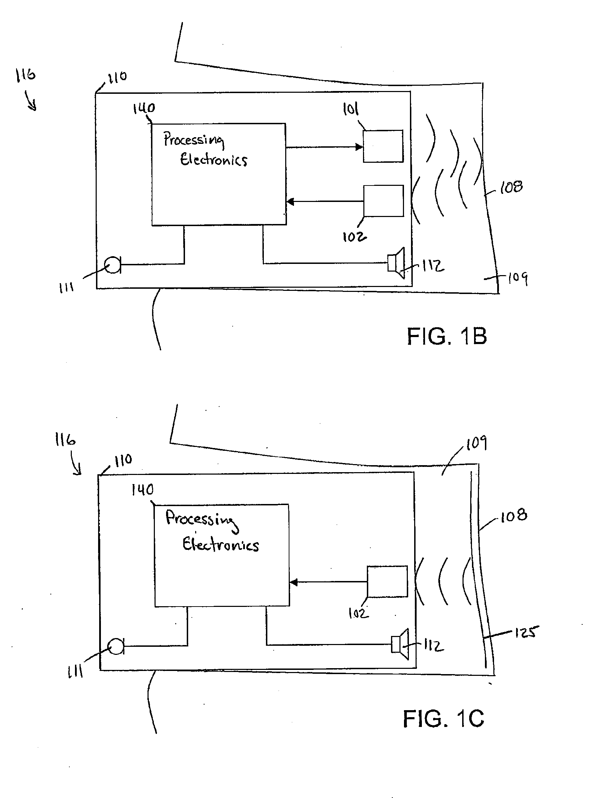

FIG. 1B is a block diagram of a hearing assistance device with ear drum vibration processing according to one embodiment of the present subject matter. The hearing assistance device 116 is adapted to be worn in a user's ear canal 109 and includes a housing 110. The illustrated embodiment shows a transducer 101 for emitting a signal toward an eardrum and a sensor 102 for receiving emitted signals reflected off the eardrum 108. The transducer 101 and sensor 102 are connected to processing electronics 140. In various embodiments, the processing electronics 140 include a processor, such as, a microprocessor or a digital signal processor (DSP), for example. In various embodiments, the processing electronics include analog components, digital components or a combination of analog and digital components. It is understood that embodiments employing analog designs and analog-digital hybrid designs may be made which fall within the scope of the present subject matter. The processing electronics determine sound pressure level and phase using the transducer 101 to generate, and the sensor 102 to receive, signals directed toward and reflected from an eardrum 108, or tympanic membrane, of a user. In various embodiments, the transducer 101 is a laser transducer and the sensor 102 is an optical sensor. The laser transducer emits laser energy toward the eardrum and the optical sensor senses reflected laser energy from a user's eardrum.
In some embodiments, a laser transducer includes a laser diode. In various embodiments, the transducer 101 is an ultrasonic emitter and the sensor 102 is an ultrasonic receiver. In various embodiments, the processing electronics 140 include hearing assistance processing. The processing electronics 140 receive a signal from a microphone 111, process the signal to assist a user's hearing and plays the processed signal to the user's ear using a speaker 112.

FIG. 1C is a block diagram of a hearing assistance device with ear drum vibration processing according to one embodiment of the present subject matter. The hearing assistance device 116 is adapted to be worn in a user's ear canal 109 and includes a housing 110. The illustrated embodiment shows a sensor 102 for generating signals corresponding to vibration of user's eardrum 108. The sensor is connected to processing electronics 140. In various embodiments, the processing electronics 140 include a processor, such as, a microprocessor or a digital signal processor (DSP), for example. In various embodiments, the processing electronics 140 include analog components, digital components or a combination of analog and digital components. It is understood that embodiments employing analog designs and analog-digital hybrid designs may be made which fall within the scope of the present subject matter. The processing electronics 140 determine sound pressure level and phase using the signal generated from the sensor 102. The signals indicative of eardrum vibration are generated by sensing changes in magnetic field strength using the sensor 102. A magnetic media 125 attached to the user's eardrum is used as a magnetic field source. In various embodiments, the processing electronics 140 include hearing assistance processing. The processing electronics 140 receive a signal from a microphone 111, process the signal to assist a user's hearing and plays the processed signal to the user's ear using a speaker 112.

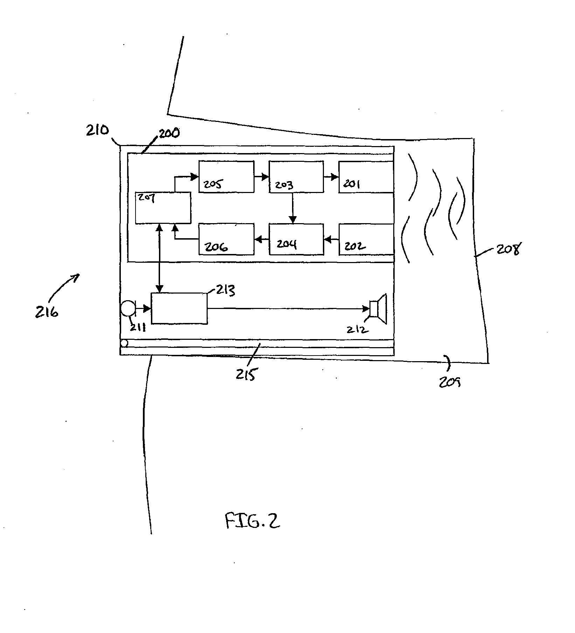

FIG. 2 illustrates a hearing assistance device 216 with an eardrum vibration detector 200 according to one embodiment of the present subject matter. FIG. 2 shows a hearing assistance device 216 including a housing 210 adapted to be worn in the ear canal 209 of a user, such as an in-the-ear (ITE) housing or a completely-in-the-canal (CIC) housing. The illustrated housing 210 includes a vent 215. The hearing assistance device 216 includes a hearing assistance processor 213 connected to a microphone 211 and a speaker 214. The hearing assistance device 216 also includes an eardrum vibration detector including a transducer 201 and driver unit 203 connected to a processor 207 using a D/A converter 205. The illustrated eardrum vibration detector also includes a sensor 202 and demodulator 204 for receiving energy reflected from the eardrum 208 and generating a signal indicative of displacement, or vibration, of the eardrum 208. In various embodiments, the transducer 201 is a laser based transducer and the sensor 202 is an optical sensor. The optical sensor receives laser energy, generated using the laser transducer, reflected from a user's eardrum to generate a signal indicative of eardrum vibration. In various embodiments, the transducer 201 is an ultrasonic transducer and the sensor 202 is an ultrasonic receiver. The ultrasonic receiver senses ultrasonic acoustic energy, generated using the ultrasonic transducer, reflected from a user's eardrum to generate a signal indicative of eardrum vibration. In the illustrated embodiment of FIG. 2, the signal indicative of eardrum displacement, or the vibration signal, is digitized using a A/D converter 206 and passed to the processor 207. The processor uses the digitized vibration signal to estimate the sound pressure level and phase at the eardrum 208. In various embodiments, the estimates provide a basis for changing parameters in the hearing assistance device to improve performance of the hearing assistance device, increase hearing comfort of the user or a combination thereof. In various embodiments, sound pressure level and phase estimates are electronically saved in the hearing assistance device for later analysis. In various embodiments, the processing electronics store vibration signal samples and parameters associated with determining sound pressure level and phase estimates.

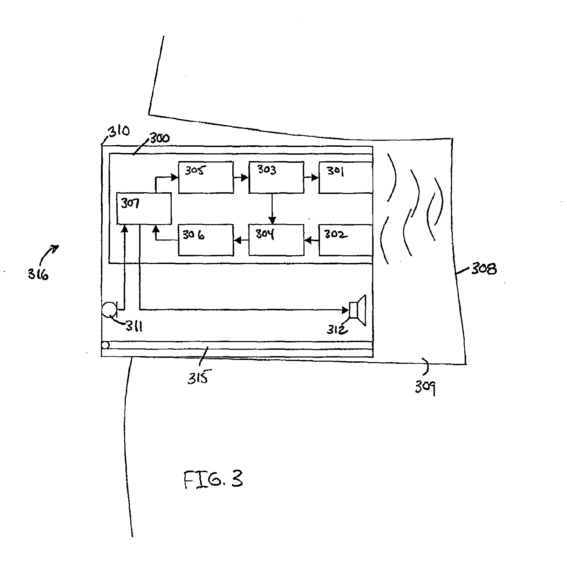

FIG. 3 illustrates a hearing assistance device 316 with an eardrum vibration detector 300 according to one embodiment of the present subject matter. FIG. 3 shows a hearing assistance device 316 including a housing 310 adapted to be worn in the ear canal 309 of a user, such as an in-the-ear (ITE) housing or a completely-in-the-canal (CIC) housing. The illustrated housing 310 includes a vent 315. The hearing assistance device 316 includes a processor 307 connected to a microphone 311 and a speaker 314. The hearing assistance device 316 also includes an eardrum vibration detector including a transducer 301 and driver unit 303 connected to the processor 307 using a D/A converter 305. The illustrated eardrum vibration detector also includes a sensor 302 and demodulator 304 for receiving energy reflected from the eardrum 308 and generating a signal indicative of displacement, or vibration, of the eardrum 308. In various embodiments, the transducer 301 is a laser transducer and the sensor 302 is a optical sensor. The optical sensor receives laser energy, generated using the laser based transducer, reflected from a user's eardrum to generate a signal indicative of eardrum vibration. In some embodiments, the laser transducer includes a laser driver. In various embodiments, the transducer 301 is an ultrasonic transducer and the sensor 302 is an ultrasonic receiver. The ultrasonic receiver senses ultrasonic acoustic energy, generated using the ultrasonic transducer, reflected from a user's eardrum to generate a signal indicative of eardrum vibration. In the illustrated embodiment of FIG. 3, the signal indicative of eardrum displacement, or the vibration signal, is digitized using an A/D converter 306 and passed to the processor 307. In the illustrated embodiment, the processor 307 includes processing for both sound pressure measurement and hearing assistance. The processor 307 uses the vibration signal to determine estimates of the sound pressure level and phase at the eardrum 308. In various embodiments, the estimates provide a basis for changing parameters in the hearing assistance device to improve performance of the hearing assistance device, increase hearing comfort of the user or a combination thereof. In various embodiments, sound pressure level and phase estimates are electronically saved in the hearing assistance device for later analysis. In various embodiments, the ear drum vibration detector 300 and the hearing assistance processing are implemented using analog components, digital components or a combination of analog and digital components.

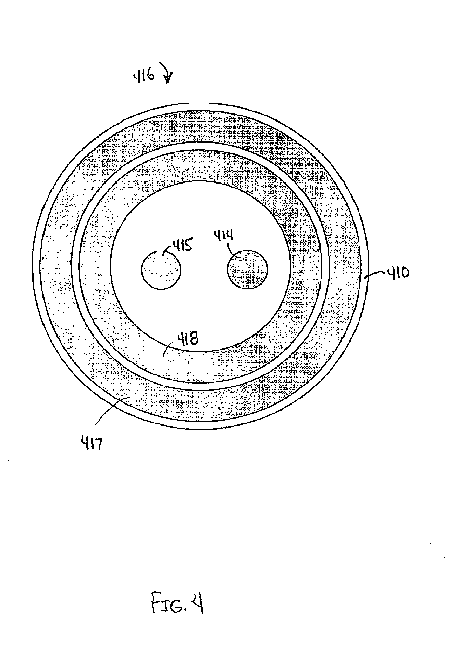

FIG. 4 illustrates an end view of a hearing assistance device 416 that supports eardrum vibration sensing according to one embodiment of the present subject matter. FIG. 4 shows the end of the hearing assistance device housing 410 including a transducer opening 417, a sensor opening 418, a vent 415 and a speaker tube 414.

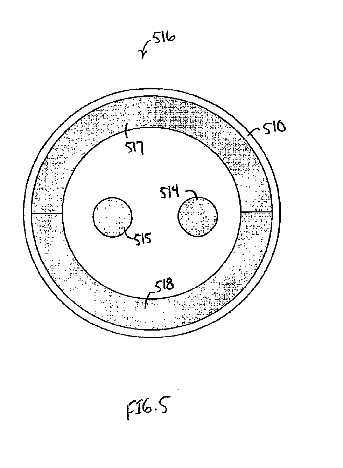

FIG. 5 illustrates an end view of a hearing assistance device 516 that supports eardrum vibration sensing according to one embodiment of the present subject matter. FIG. 5 shows the end of the hearing assistance device housing including a transducer opening 517, a sensor opening 518, a vent 515 and a receiver tube 514.

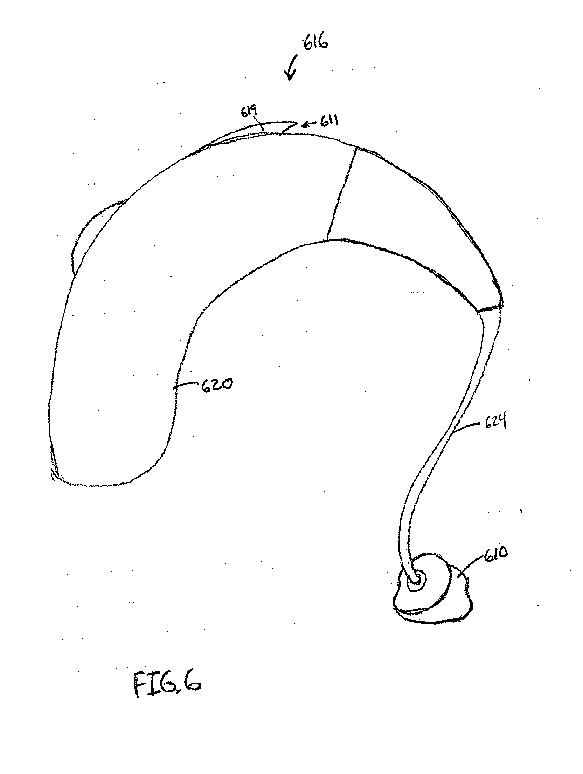

FIG. 6 illustrates a hearing assistance device 616 having a behind-the-ear (BTE) housing 620 that supports ear drum vibration sensing according to one embodiment of the present subject matter. FIG. 6 shows a BTE housing 620, including a microphone hood 619, an ear mold 610 and a cable assembly 624 connecting the earmold 610 to the BTE housing 620. In various embodiments, the hearing assistance device includes a transducer and a sensor for detecting eardrum vibration. In various embodiments, the cable assembly is adapted to transmit signals between the ear mold and the BTE housing for eardrum vibration detection and processing. The BTE housing 620 includes hearing assistance electronics, such as a microphone and a processor. In the illustrated embodiment of FIG. 6, the ear mold includes a speaker and mounting apparatus to retain the cable assembly. The speaker emits acoustical signal at the user's eardrum. In various embodiments, the hearing assistance electronics connect to the receiver in the ear mold using wires forming at least a portion of the cable 624 connecting the BTE housing 620 to the ear mold 610.

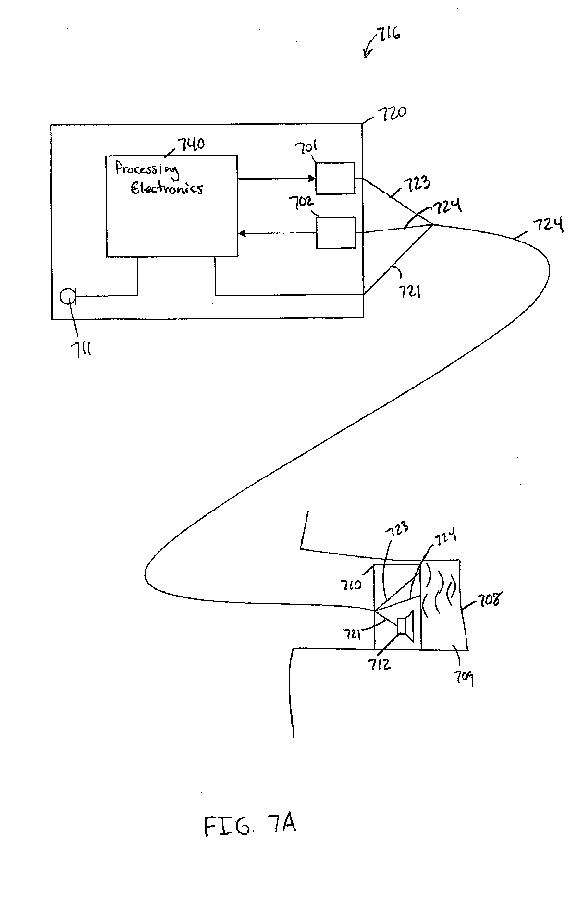

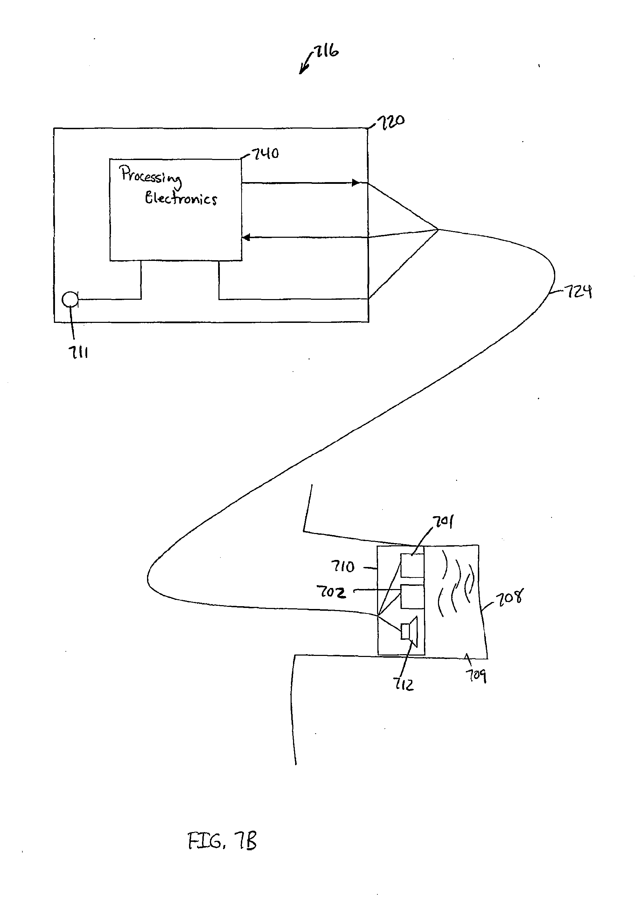

FIG. 7A illustrates a block diagram of a hearing assistance device 716 having a behind-the-ear (BTE) housing 720 that supports ear drum vibration sensing according to one embodiment of the present subject matter. FIG. 7 shows a BTE housing 720, a cable assembly 724 and a second housing 710 including a speaker 712 to be worn in the ear canal 709 of a user. In various embodiments, the second housing 710 is an ear mold, for example, or an ear bud. In the illustrated embodiment, the second housing 710 includes a speaker 712 and fiber optics 723, 724 for emitting and receiving optical energy, such as laser light, for detecting eardrum vibration. The second housing 710 uses a cable assembly 724 to connect to the BTE housing 720. In various embodiments, the cable assembly 724 includes fiber optics for transmitting laser energy between the second housing 710 and the BTE housing 720. In the illustrated embodiment, the cable assembly 724 includes an emission fiber cable 723 for transmitting light from the laser source 701 to the second housing 710 and a reception fiber cable 724 for transmitting laser light from the second housing 710 to the sensor 702. In various embodiments, the cable assembly 724 includes conductors 721 for connecting hearing assistance electronics located in the BTE housing 720 with a speaker 712 coupled to the second housing 710. In some embodiments, the laser source includes a laser driver. IN various embodiments, the laser source includes a low level laser diode.

In the illustrated embodiment, the BTE housing 720 includes a microphone 711, processing electronics 740, a transducer 701 and a sensor 702. The processing electronics control detection and data analysis of signals indicative of eardrum vibration. In the illustrated embodiment, the transducer is a laser light source 701 and the sensor is an optical sensor. Eardrum reflected laser light received using the optical sensor is used to detect eardrum vibration and for analysis and estimation of the sound field at the eardrum 708. In various embodiments, the estimates provide a basis for changing parameters in the hearing assistance device to improve performance of the hearing assistance device, increase hearing comfort of the user or a combination thereof. In various embodiments, sound pressure level and phase estimates are electronically saved in the hearing assistance device for later analysis. In various embodiments, the laser source is enclosed in the ear mold and connected to the processing electronics in the BTE using conductors in the cable assembly. In various embodiments the sensor 702 includes a demodulator to decrease the processing load of the processing electronics 740. In some embodiments the demodulator is coupled to the processing electronics, the laser source and the sensor. In various embodiments the processing electronics include a digital signal processor (DSP).

FIG. 7B illustrates a block diagram of a hearing assistance device 716 having a behind-the-ear (BTE) housing 720 that supports ear drum vibration sensing according to one embodiment of the present subject matter. FIG. 7 shows a BTE housing 720, a cable assembly 724 and a second housing 710 including a speaker 712 to be worn in the ear canal 709 of a user. In various embodiments, the second housing 710 is an ear mold, for example, or an ear bud. In the illustrated embodiment, the second housing 710 includes a receiver 712, a transducer 701 and a sensor 702 emitting and receiving acoustic energy, such as ultrasonic sound waves, for detecting eardrum vibration. The second housing 710 uses a cable assembly 724 to connect to the BTE housing 720. In various embodiments, the cable assembly 724 includes conductors for connecting the processing electronics 740 in the BTE housing to the speaker, transducer and the sensor.
In the illustrated embodiment, the BTE housing 720 includes a microphone 711 and processing electronics 740. The processing electronics 740 control detection and data analysis of signals indicative of eardrum vibration. In the illustrated embodiment, the transducer 701 is an ultrasonic emitter and the sensor 702 is an ultrasonic receiver. Eardrum reflected ultrasonic sound received using the ultrasonic receiver is used to detect eardrum vibration and for analysis and estimation of the sound field at the eardrum 708. In various embodiments, the sound field estimates provide a basis for changing parameters in the hearing assistance device to improve performance of the hearing assistance device, increase hearing comfort of the user or a combination thereof. In various embodiments, sound pressure level and phase estimates are electronically saved in the hearing assistance device for later analysis.

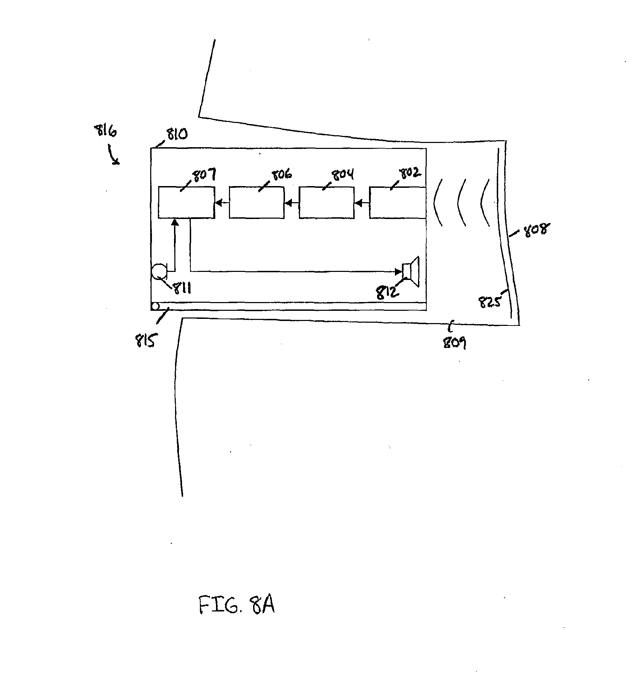

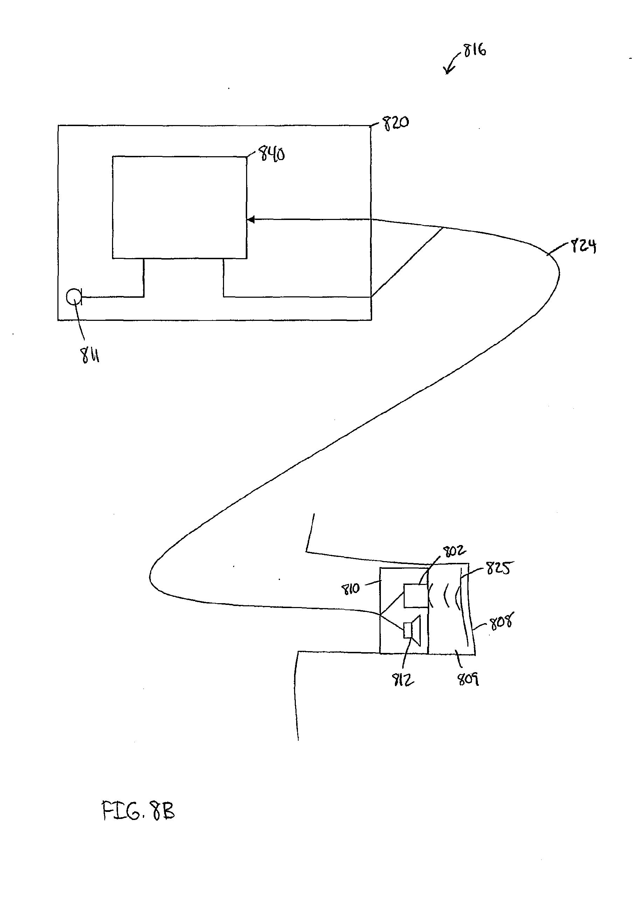

FIG. 8A illustrates a hearing assistance device 816 using magnetic wave detection electronics to estimate sound field at the eardrum 808 of a user according to one embodiment of the present subject matter. FIG. 8 shows a hearing assistance housing 810 positioned in the ear canal 809 of a user. The housing 810 includes a processor 807, hearing assistance electronics and eardrum vibration detection electronics. The hearing assistance electronics include a microphone 811 and a receiver 812. In the illustrated embodiment, the hearing assistance device housing 810 includes a receiver tube 814 to direct sound from the receiver toward the user's eardrum. In the illustrated embodiment, the housing 810 includes a vent 815 to minimize complete occlusion of the user's ear canal.

The eardrum vibration detection electronics include a magnetic wave sensor 802, such as a coil, an amplification unit 804 and an A/D converter 806 for connecting the sensor output to the processor 807. In various embodiments, the hearing assistance device includes more than one processor to process sound and estimate the sound field at the user's eardrum. Also illustrated in the embodiment of FIG. 8, is a magnetic material 825 attached to the user's tympanic membrane, or eardrum 808. A thin magnet and a magnetic film are examples magnetic material used for attaching to the user's ear drum.

The magnetic material 825 produces a magnetic field near the eardrum 808 of the user. The magnetic material 825 vibrates with the eardrum 808 and induces change in the magnetic field near the eardrum including the magnetic field in the ear canal 809. A change in magnetic field intensity will induce a signal in a coil present in and properly orientated to the magnetic field. In various embodiments, the amplification electronics 804 include electronics to process the signal generated by the coil 802 in the changing magnetic field within a user's ear canal 809.

FIG. 8B illustrates a block diagram of a hearing assistance device 816 having a behind-the-ear (BTE) housing 820 that supports ear drum vibration sensing according to one embodiment of the present subject matter. FIG. 8B shows a BTE housing 820, a cable assembly 824 and a second housing 810 including a speaker 812 to be worn in the ear canal 809 of a user. In various embodiments, the second housing 810 is an ear mold, for example, or an ear bud. In the illustrated embodiment, the second housing 810 includes a receiver 812 and a magnetic sensor 802 such, as a coil sensor, for detecting eardrum vibration. The second housing 810 uses a cable assembly 824 to connect to the BTE housing 820. The cable assembly 824 includes conductors for connecting devices enclosed in the second housing 810 to the processing electronics 740 in the BTE housing 820.

In the illustrated embodiment, the BTE housing 820 includes a microphone 811 and processing electronics 840. The processing electronics control detection and data analysis of signals received using the magnetic sensor 802 and indicative of eardrum vibration. The magnetic sensor 802 senses changes in a magnetic field established using magnetic media 825 attached to the user's eardrum 808. Signals indicative of eardrum vibration are used for analysis and estimation of the sound field at the eardrum 808. In various embodiments, the sound field estimates provide a basis for changing parameters in the hearing assistance device to improve performance of the hearing assistance device, increase hearing comfort of the user or a combination thereof. In various embodiments, sound pressure level and phase estimates are electronically saved in the hearing assistance device for later analysis. In various embodiments the processing electronics include one or more processors, including one or more DSPs.

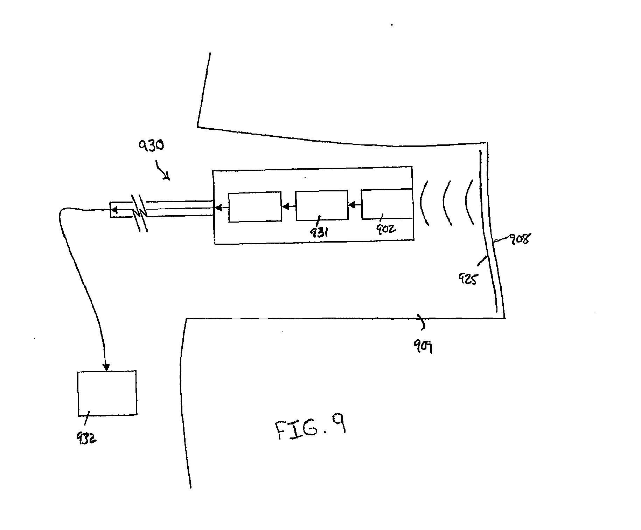

FIG. 9 illustrates a magnetic wave probe 930 for detecting eardrum vibration of a user and estimating the sound field at the user's eardrum according to one embodiment of the present subject matter. The probe 930 senses variation in magnetic field intensity using a coil 902 mechanically coupled to the probe 930. A magnetic material 925, such as a thin magnet or a magnetic film, attaches to the user's eardrum 908. The magnetic material 925 provides a magnetic field about the eardrum 908 of the user. The magnetic material 925 vibrates with the eardrum 908 and induces change in the magnetic field about the eardrum 908 including the magnetic field in the ear canal 909.
A change in magnetic field intensity will induce a signal in a coil present and properly orientated in the magnetic field. In various embodiments, the probe coil connects to amplification electronics 931. In various embodiments, the amplification electronics 931 include electronics to process a signal generated by the coil in the changing magnetic field within the ear canal of a user. In the illustrated embodiment, amplification electronics 931 connect the coil signal to an A/D converter 906 for digitizing the signal for processing using a connected, remote processor 932. The signal includes indications of the movement of the tympanic membrane in response to various acoustic waves. The processor uses the signal to estimate the sound field at the tympanic membrane. In various embodiments, the estimates provide a basis for setting or changing parameters in a hearing assistance device to improve performance of the hearing assistance device, increase hearing comfort for the user of the device or a combination thereof. In various embodiments, sound pressure level and phase estimates are electronically saved in the remote processor for later analysis.

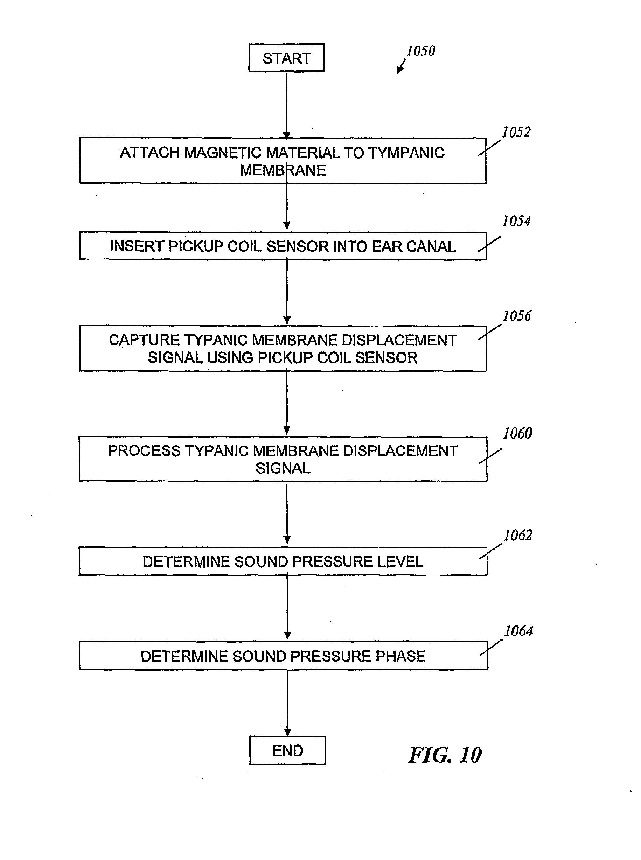

FIG. 10 illustrates a flow diagram 1050 for estimating sound pressure level and phase at the eardrum of a user according to the present subject matter. The method 1050 includes attaching a magnetic material to the user's tympanic membrane, or eardrum 1052, inserting a pickup coil sensor into user's ear canal adjacent the tympanic membrane 1054, capturing the coil signal indicative of movement or displacement of the tympanic membrane 1056, processing the signal indicative of movement or displacement of the tympanic membrane 1058 and determining the sound pressure level 1060 and phase 1062 at the tympanic membrane.

The present subject matter includes hearing assistance devices, including, but not limited to, hearing aids, such as behind-the-ear (BTE), in-the-ear (ITE), in-the-canal (ITC), or completely-in-the-canal (CIC) type hearing aids. It is understood that behind-the-ear type hearing aids may include devices that reside substantially behind the ear or over the ear. Such devices may include hearing aids with receivers associated with the electronics portion of the behind-the-ear device, or hearing aids of the type having receivers in-the-canal. It is understood that other hearing assistance devices not expressly stated herein may fall within the scope of the present subject matter.

This application is intended to cover adaptations and variations of the present subject matter. It is to be understood that the above description is intended to be illustrative, and not restrictive. The scope of the present subject matter should be determined with reference to the appended claims.

## Claims

1. A device (116) for measuring sound pressure near a user's tympanic membrane, the device (116) comprising:
a vibration detector (101,102) operable to detect vibrations of a user's tympanic membrane and generate a signal indicative of the detected movement of the tympanic membrane wherein the vibration detector (101, 102) comprises a source (101) operable to generate a signal which varies dependent upon the movement of the tympanic membrane and a sensor (102) operable to detect the signal generated by the source (101);
a processor (140) adapted to estimate a value related to the movement of the tympanic membrane using the signal received from the sensor(102); and
a housing (110) enclosing the vibration detector (101,102).

2. The device of claim 1 wherein the vibration detector (101,102) comprises:
a magnetic field source (101) coupled to the user's tympanic membrane;
a sensor (102) adapted to detect the magnetic field source and generate a signal indicative of movement of the tympanic membrane.

3. The device of claim 2, wherein the magnetic field source includes a thin magnet.

4. The device of claim 2 or claim 3, wherein the magnetic field source includes a magnetic film.

5. The device of claim 1 wherein the vibration detector (101,102) comprises:
a laser source (101) adapted to project a beam of laser energy at a user's tympanic membrane; and
a sensor (102) for receiving reflected laser energy directed at the tympanic membrane.

6. The device of claim 1 wherein the vibration detector (101,102) comprises:
an ultrasonic wave source (101) adapted to project ultrasonic waves at a user's tympanic membrane;
an ultrasonic sensor (102) for receiving reflected ultrasonic waves directed at the tympanic membrane.

7. The device of any of claims 1-6, wherein the value includes a sound pressure level or a sound pressure phase.

8. The device of any of claims 1-7, further comprising a microphone (111) coupled to the processor (140).

9. The device of any of claims 1-8, further comprising a speaker (112) coupled to the processor (140).

10. A method of estimating sound pressure in an ear canal of a user, the method comprising:
providing a vibration detector (101,102) operable to detect vibration of the tympanic membrane of a user's ear wherein the vibration detector (101, 102) comprises a source (101) operable to generate a signal which varies dependent upon the movement of the tympanic membrane and a sensor (102) operable to detect the signal generated by the source (101);
detecting movement of the tympanic membrane using the vibration detector (101,102);
generating a vibration signal indicative of the detected movement of the tympanic membrane; and
estimating a value related to sound in the ear canal using the vibration signal.

11. The method of claim 10 wherein providing a vibration detector (101,102) operable to detect vibration of the tympanic membrane of a user's ear comprises providing a magnetic field source (101) disposed on user's tympanic membrane and wherein detecting movement of the tympanic membrane comprises detecting magnetic field variations arising due to movement of the magnetic field source using a magnetic sensor (102) disposed in the ear canal.

12. The method of claim 10 wherein providing a vibration detector (101,102) operable to detect vibration of the tympanic membrane of a user's ear comprises providing a laser source (101) adapted to project a beam of laser energy at a user's tympanic membrane, and wherein detecting movement of the tympanic membrane comprises detecting variations in laser energy reflected by the tympanic membrane arising due to movement of the tympanic membrane.

13. The method of claim 10 wherein providing a vibration detector (101,102) operable to detect vibration of the tympanic membrane of a user's ear comprises providing a ultrasound source (101) adapted to project ultrasonic waves at a user's tympanic membrane, and wherein detecting movement of the tympanic membrane comprises detecting variations in ultrasonic waves reflected by the tympanic membrane arising due to movement of the tympanic membrane.

14. The method of any of claims 10-13, wherein estimating a value includes estimating sound pressure level and/or sound pressure phase near the tympanic membrane.

15. The method of any of claims 10-14, wherein estimating a value includes adjusting a hearing assistance device parameter.
